# EUROPÄISCHE PATENTANMELDUNG

(11) **EP 0 524 616 A1**
(43) Veröffentlichungstag der Anmeldung: **27.01.1993**
(21) Anmeldenummer: 92112504.3
(22) Anmeldetag: 22.07.1992
(51) Int. Cl.: A61M 5/14

(54) **Flaschenständer-Kopf**

(30) Priorität: 24.07.1991 DE 4124563
(71) Anmelder: Carl-Jürgen Voss, D-90453 Nürnberg (DE)
(72) Erfinder: Carl-Jürgen Voss, D-90453 Nürnberg (DE)
(74) Vertreter: von Hellfeld, Axel, Dr. Dipl.-Phys.

(57) **Zusammenfassung**

Ein Flaschenständer-Kopf zum Halten einer oder mehrerer Flaschen, die mit einer Lösung befüllt sind, welche in einen menschlichen Körper zu überführen ist, weist eine Abstützung (12) auf, die der Flaschenform angepaßt ist und mit dem Flaschenständer-Kopf (10) verbunden ist.

## Beschreibung

Die Erfindung bezieht sich auf einen Flaschenständer-Kopf zum Halten einer oder mehrerer mit in einen menschlichen Körper zu überführender Lösung gefüllter Flaschen, wobei die Flaschen derart gehalten werden, daß ihre Öffnungen im wesentlichen senkrecht nach unten weisen.

Flaschenständer mit solchermaßen ausgebildeten Köpfen werden zumeist in Krankenhäusern und Arztpraxen eingesetzt. Sie dienen zur Halterung von Flaschen oder Beuteln mit Infusions-, Transfusions- und Diätetika-Lösungen sowie Lösungen für die enterale und parenterale Ernährung. Die Flaschen sind kopfüber am Flaschenständer gehalten, so daß ein Ende eines Schlauches mit der unten liegenden Öffnung der Flasche verbunden werden kann, dessen anderes Ende in den Körper eines Patienten geführt ist. Auf diese Weise wird der Inhalt der Flasche mittels eines Infusionsgerätes in den Körper des Patienten überführt.

Um die Flaschen am Flaschenständer anbringen zu können, sind üblicherweise spezielle, meist aus Kunststoff bestehende Aufhänger z. B. in Spiral- oder Bügelform vorgesehen, mit denen die Flasche an dem Flaschenständer-Kopf aufgehängt wird. Zu diesem Zweck weisen bekannte Flaschenständer zumindest einen, meist jedoch mehrere Haken auf, an die die mit dem Aufhänger versehenen Flaschen gehängt werden, so daß ihre Austrittsöffnung nach unten weist. Flaschenständer der beschriebenen Art können sowohl fahrbar sein, um einem beispielsweise an einem Tropf hängenden Patienten eine gewisse Mobilität zu ermöglichen, als auch fest mit einem Krankenbett verbunden sein. Die letztgenannte Ausführung ist auch unter der Bezeichnung Bettgalgen bekannt.

Da die zur Anbringung am Flaschenständer dienenden Aufhänger nur einmal verwendet werden können, ist es aus Kostengründen sowie aus Gründen der Abfallproblematik wünschenswert, auf solche Aufhänger zu verzichten. Herkömmliche Flaschenständer sind jedoch nicht dazu geeignet, Flaschen ohne Aufhänger in der gewünschten, mit der Austrittsöffnung nach unten weisenden Position zu halten.

Aus dem DE-GM 89 09 304.6 ist eine Vorrichtung zum Ausrichten einer ersten Flasche in bezug auf eine zweite Flasche bekannt. Diese Vorrichtung weist zwei starr miteinander verbundene und koaxial zueinander angeordnete Aufnahmen auf, in die je eine Flasche mit ihrem Hals einführbar ist. Die Aufnahmen weisen je drei freistehende, einstückig ausgebildete Arme auf, die sich im wesentlichen in axialer Richtung der Vorrichtung erstrecken. Zweck der Vorrichtung ist es, das Mischen zweier in unterschiedlichen Flaschen enthaltenen Komponenten einer Lösung zu vereinfachen.

Aus der Druckschrift Siemens-Elema AB: Druckwandlerstativ 448/3, Prospekt E 563, Medicinsk Teknik, Solna Sweden, PA 107513, 1975 ist eine Haltevorrichtung für eine Infusions- oder Transfusionsflasche bekannt. Auch aus der DE-GM 75 10 285, DE-GM 73 33 063 und der US 4 030 690 sind Haltevorrichtungen für Infusions- bzw. Transfusionsflaschen bekannt.

Der vorstehend zitierte Stand der Technik lehrt aber nicht, mehrere Abstützungen an einem Flaschenständer-Kopf winkelmäßig versetzt fest anzubringen.

Der Erfindung liegt die Aufgabe zugrunde, einen Flaschenständer-Kopf zu schaffen, bei dem zum Halten der Flaschen in einer Position, in der ihre Austrittsöffnung nach unten gerichtet ist, keine Aufhänger mehr benötigt werden. Der Flaschenständer-Kopf soll gegen herkömmliche Köpfe auswechselbar sowie kostengünstig in der Herstellung sein und zudem die Möglichkeit bieten, Flaschen unterschiedlicher Größe zu halten.

Erfindungsgemäß ist die Aufgabe durch einen Flaschenständer-Kopf gelöst, bei dem jede Flasche in einer der Flaschenform angepaßten und mit dem Flaschenständer-Kopf verbundenen Abstützung aufgenommen ist.

In einer vorteilhaften Ausführungsform weist die Abstützung einen den Flaschenhals aufnehmenden Ring und zumindest einen den Flaschenkörper wenigstens teilweise umgreifenden Arm auf. Der Arm kann beispielsweise schraubenfederförmig ausgeführt sein.

In einer anderen Ausführungsform sind an dem Ring drei sich im wesentlichen vertikal nach oben erstreckende Arme winkelmäßig versetzt befestigt.

In einer produktionstechnisch besonders einfach herzustellenden Ausführungsform ist die Abstützung durch eine Grundplatte mit zumindest einer einen Flaschenhals aufnehmenden kreisförmigen Ausnehmung und zumindest einem einen Flaschenkörper wenigstens teilweise umgreifenden Arm gebildet. Dabei entspricht die Zahl der Ausnehmungen der Anzahl Flaschen, die von dem Flaschenständer-Kopf gehalten werden sollen. Vorteilhaft erstrecken sich je drei Arme, die winkelmäßig versetzt um eine kreisförmige Ausnehmung herum angeordnet sind, im wesentlichen vertikal nach oben.

Bei allen Ausführungsformen sind der oder die Arme bevorzugt einstückig ausgebildet. In einer abgewandelten Ausführungsform sind der oder die Arme federnd nachgiebig ausgebildet.

Soll der Flaschenständer-Kopf mehrere Flaschen aufnehmen, sind die entsprechenden Abstützungen bevorzugt in einer rechtwinkelig zur Vertikalachse des Flaschenständers liegenden Ebene winkelmäßig versetzt angeordnet. Der Winkelversatz sowie der Abstand der Abstützungen zur Vertikalachse wird dabei vorteilhaft so gewählt, daß auch ein nicht vollständig mit Flaschen bestückter Ständer eine möglichst geringe Neigung zum Umkippen zeigt.

Alle Ausführungsformen des Flaschenständer-Kopfes weisen bevorzugt einen oder mehrere Haken auf, um auch das Aufhängen von noch im Umlauf befindlichen herkömmlichen Flaschen mit Aufhängern oder von beispielsweise Infusionsbeuteln zu gestatten.

Der erfindungsgemäße Flaschenständer-Kopf ist so gestaltet, daß er auf herkömmliche Flaschenständer aufschraub- oder aufsteckbar und deshalb gegen bekannte Flaschenständer-Köpfe auswechselbar ist.

Die Erfindung wird im folgenden anhand schematischer Zeichnungen näher erläutert. Es zeigt:
- Fig. 1: eine räumliche Darstellung einer ersten Ausführungsform eines Flaschenständer-Kopfes in einer Ansicht von schräg oben;
- Fig. 2: eine zweite Ausführungsform des Flaschenständer-Kopfes, ebenfalls in räumlicher Darstellung und von schräg oben gesehen;
- Fig. 3: die Draufsicht der Ausführungsform gemäß Fig. 1. und
- Fig. 4: und 5 weitere Ausführungsformen eines Flaschenständers-Kopfes.

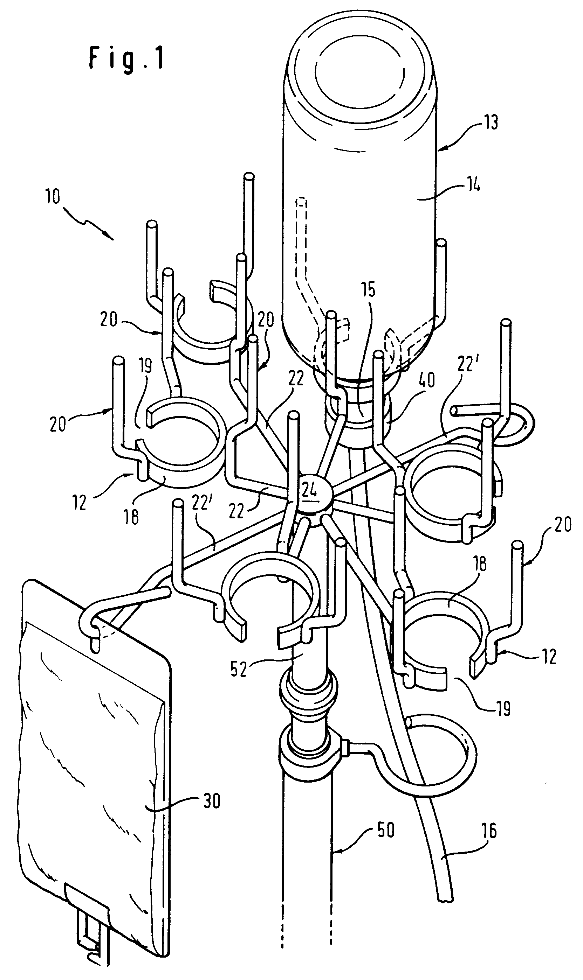

Ein Flaschenständer-Kopf 10 weist sechs Abstützungen 12 zur Aufnahme einer Flasche 13 auf. Wie aus Fig. 1 ersichtlich, ist die Flasche 13 dabei so aufgenommen, daß ihre Öffnung, die mit einem Schlauch 16 verbunden ist, annähernd senkrecht nach unten weist. Jede Abstützung 12 weist einen Ring 18 auf, an dessen Außenseite drei Arme 20 jeweils um 120° versetzt befestigt sind, die sich im wesentlichen vertikal nach oben erstrecken. Der Ring 18 ist nicht ganz geschlossen, sondern weist einen Durchlaß 19 auf, damit die Flasche 13 mit angeschlossenem Schlauch 16 problemlos in die Abstützung 12 gesetzt oder aus ihr genommen werden kann. Der Durchmesser des Rings 18 ist größer als der Durchmesser des Flaschenhalses 15 bzw. eines Verschlusses 40 der Flaschenauslaßöffnung, jedoch kleiner als der Durchmesser des Flaschenkörpers 14 gewählt.

Die Arme 20 sind der Außenkontur der Flasche 13 zumindest annähernd angepaßt. Der Arm 20 jeder Abstützung 12, der der Vertikalachse eines in Fig. 1 angedeuteten Flaschenständers 50 am nächsten ist, ist einstückig mit einem Schenkel 22 verbunden, dessen anderes Ende an einem zentralen Aufnahmeteil 24 befestigt ist. Die Abstützungen 12 sind also im wesentlichen kreisförmig um das Aufnahmeteil 24 herum angeordnet. Das Aufnahmeteil 24 ist in ein Rohr 52 des Flaschenständers 50 eingeschraubt oder eingesteckt.

Zusätzlich zu den sechs Abstützungen 12 sind am zentralen Aufnahmeteil 24 zwei Schenkel 22' befestigt, die länger als die Schenkel 22 sind und deren nach außen weisendes Ende hakenförmig gebogen ist. Wie in Fig. 1 gezeigt, kann an das hakenförmig gebogene Ende der Schenkel 22' beispielsweise ein Infusionsbeutel 30 gehängt werden.

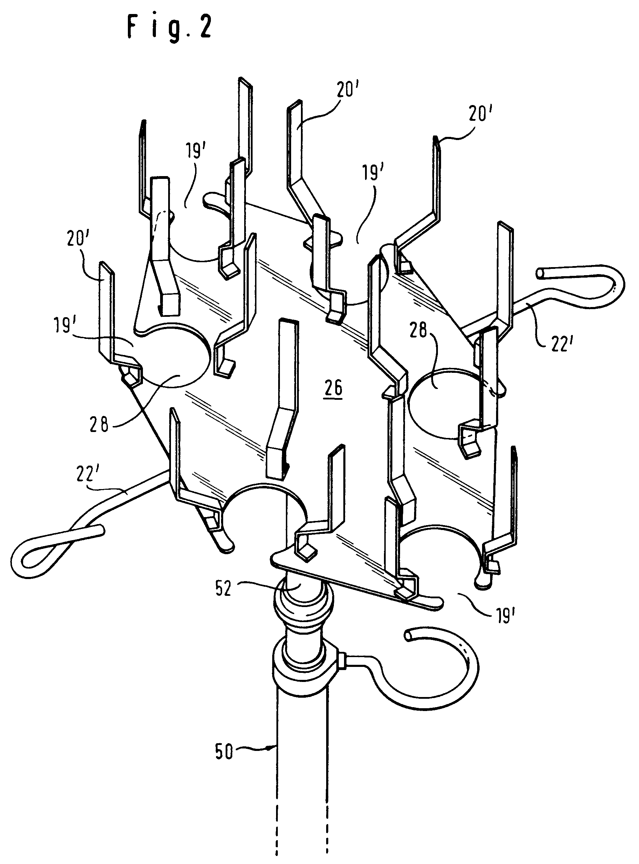

Fig. 2 zeigt eine zweite Ausführungsform des Flaschenständer-Kopfes 10. Eine einteilige Grundplatte 26 ersetzt die Ringe 18 und die Schenkel 22 der in Fig. 1 gezeigten Ausführungsform. Die Grundplatte 26 ist in der Mitte mit dem Rohr 52 des Flaschenständers 50 verbunden und weist sechs kreisförmige Ausnehmungen 28 auf. Die Ausnehmungen 28 sind im Randbereich der Grundplatte 26 angeordnet und weisen je einen Durchlaß 19' auf, der dem Durchlaß 19 aus Fig. 1 entspricht. Der Durchmesser der Ausnehmungen 28 ist so gewählt, daß er größer als der Durchmesser des Flaschenhalses 15 bzw. eines Verschlusses 40 und kleiner als der Durchmesser des Flaschenkörpers 14 ist.

Um jede Ausnehmung 28 herum sind etwa um 120° versetzt drei Arme 20' angeordnet, die mit der Grundplatte 26 z. B. durch Schweißen verbunden sind und sich im wesentlichen vertikal nach oben erstrecken. Abweichend vom runden Querschnitt der Arme 20 (s. Fig. 1) ist der Querschitt der Arme 20' rechteckig. Analog zu den Armen 20 sind auch die Arme 20' der äußeren Kontur der Flasche 13 annähernd angepaßt und umgreifen diese zum Teil.

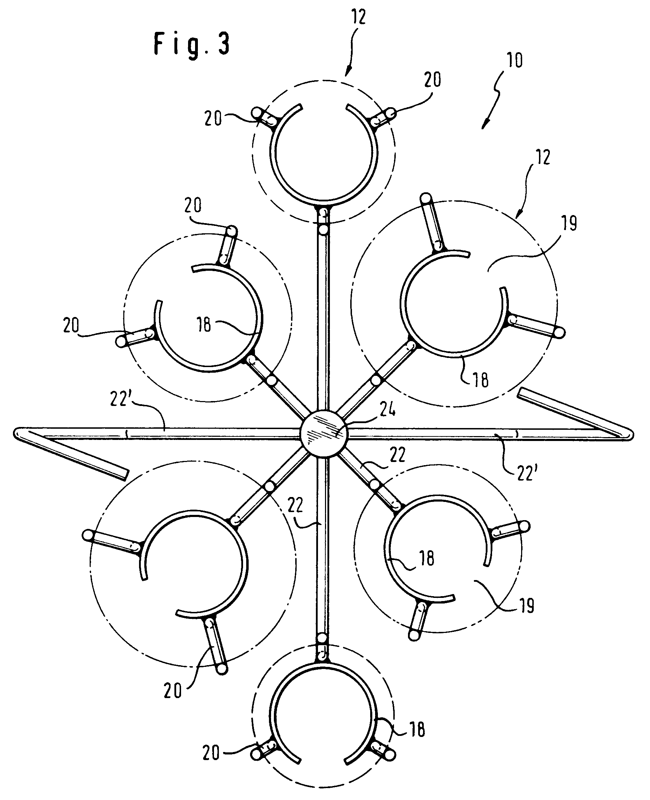

Aus der die Draufsicht der Ausführungsform gemäß Fig. 1 zeigende Fig. 3 ist zu ersehen, daß der von den Armen 20 umgrenzte zylinderförmige Raum, in Fig. 3 angedeutet durch gestrichelte bzw. strichpunktierte Linien, nicht bei allen Abstützungen 12 gleich groß ist. Gemäß Fig. 3 können somit Flaschen dreier unterschiedlicher Durchmesser in dem Flaschenständer-Kopf 10 gehalten werden. Bei sich bezüglich des zentralen Aufnahmeteils gegenüberliegenden Abstützungen 12 ist der von den Armen 20 umgrenzte Raum jeweils gleich groß.Die Schenkel 22 und 22' sind am zentralen Aufnahmeteil 24 um je 45° bezüglich des nächstfolgenden Schenkels in einer rechtwinkelig zur Vertikalachse des Flaschenständers 50 liegenden Ebene versetzt angeordnet.

Der Durchmesser des von den Armen 20 und 20' umgrenzten zylinderförmigen Raumes ist etwas kleiner als der Durchmesser des entsprechenden Flaschenkörpers 14, sodaß beim Einschieben der Flasche 13 in die Abstützung 12 die Arme 20, 20' leicht gespreizt werden und die Flasche einklemmen. Statt der Arme 20, 20' ist auch die Verwendung zweier einer entsprechenden Flasche 13 angepaßten Halbschalen möglich.

Sowohl die in Fig. 1 und Fig. 3 gezeigte erste als auch die in Fig. 2 gezeigte zweite Ausführungsform kann aus Metall, beispielsweise Stahl oder auch Aluminium, oder aus einem geeigneten Kunststoff hergestellt sein.

Der Vorteil der Flaschenständer-Köpfe besteht u. a. auch darin, daß ein herkömmlicher Ständer (50) umgerüstet werden kann. Hierzu braucht auf den Ständer (50) nur der neue Flaschenständer-Kopf aufgesetzt zu werden, z.B. durch eine Gewindeverbindung.

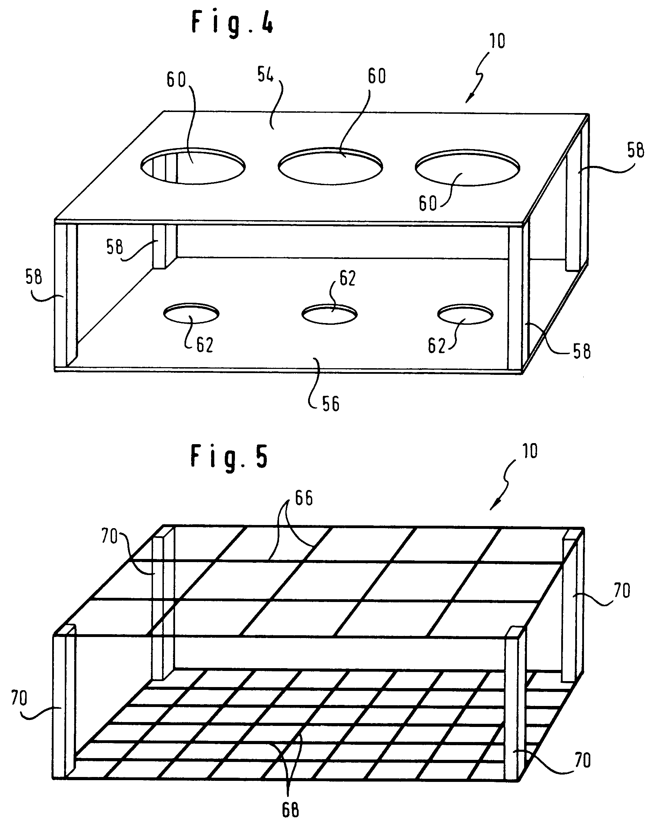

Die Fig. 4 und 5 zeigen weitere Ausführungsbeispiele eines Flaschenständer-Kopfes, die sich durch eine besonders einfache und kostengünstige Herstellung auszeichnen.

Beim Ausführungsbeispiel gemäß Fig. 4 sind zwei Platten 54, 56, z.B. aus Blech oder Kunststoff, vertikal übereinander angeordnet. Die Platten erstrecken sich parallel zueinander in horizontaler Richtung. Es versteht sich, daß die Begriffe vertikal, horizontal, oben und unten in dieser Anmeldung in bezug auf die Schwerkraft zu verstehen sind. In den Platten 54, 56 sind jeweils eine Mehrzahl von Löchern 60, 62 ausgeformt. In der oberen Platte 54 haben die Löcher 60 einen größeren Durchmesser als in der unteren Platte 56. Löcher 60 der oberen Platte 54 und Löcher 62 der unteren Platte 56 sind jeweils paarweise einander zugeordnet derart, daß die Mittellinien jeweils zweier Löcher zusammenfallen und sich vertikal erstrecken. Der Durchmesser der Löcher 60 in der oberen Platte 54 ist so bemessen, daß die Löcher genau einen Flaschenkörper aufnehmen, während der Durchmesser der Löcher 62 in der unteren Platte 56 so bemessen ist, daß gerade ein Flaschenhals hindurchpaßt und sich die Schulter der Flasche somit von oben auf der Platte 56 abstützt. Streben 58 halten die beiden Platten 54, 56 unter dem gewünschten Abstand, der etwas kürzer ist als die Längserstreckung eines Flaschenkörpers 14.

Fig. 5 zeigt eine Variante des Flaschenständer-Kopfes 10, die eine besonders einfache Herstellung ermöglicht. Statt der im Ausführungsbeispiel gemäß Fig. 4 vorgesehenen zwei Platten sind nunmehr zwei Gitter 66, 68 vorgesehen, wobei der Abstand der einzelnen Streben des oberen Gitters 66 größer ist als der Abstand der Streben des unteren Gitters 68. Die Gitterabstände sind so bemessen, daß ein Flaschenkörper gerade in eine viereckige Öffnung des oberen Gitters 66 paßt, während eine viereckige Öffnung des unteren Gitters 68 gerade einen Flaschenhals aufnehmen kann und sich somit die Flasche mit ihrer Schulter auf dem unteren Gitter 68 abstützen kann. Wie beim Ausführungsbeispiel gemäß Fig. 4 wird also die Flasche kopfüber von oben nach unten eingeführt. Beim Ausführungsbeispiel gemäß Fig. 5 sind die Gitter 66, 68 so zueinander ausgerichtet, daß jeweils zwei viereckige Öffnungen in vertikaler Richtung eine gemeinsame Mittelachse aufweisen (ebenso wie beim Ausführungsbeispiel gemäß Fig. 4).

Die Mittel, mit denen die Flaschenständer-Köpfe 10 gemäß den Fig. 4 und 5 auf einem Ständer 50 befestigbar sind, sind der Einfachheit halber weggelassen.

## Patentansprüche

1. Flaschenständer-Kopf (10) zum Halten von mit in einen menschlichen Körper zu überführender Lösung gefüllten Flaschen (13) so, daß deren Öffnung im wesentlichen senkrecht nach unten weist,
**gekennzeichnet** durch mehrere der Flaschenform angepaßte und mit dem Flschaneständer-Kopf (10) fest verbundene Abstützungen (12; 60, 62; 66, 68), die in einer zur Vertikalachse des Flaschenständers (50) horizontalen Ebene winkelmäßig versetzt angeordnet sind.

2. Flaschenständer-Kopf nach Anspruch 1,
dadurch **gekennzeichnet,** daß die Abstützung (12) einen den Flaschenhals (15) aufnehmenden Ring (18) und zumindest einen den Flaschenkörper (14) wenigstens teilweise umgreifenden Arm (20) aufweist.

3. Flaschenständer-Kopf nach Anspruch 2,
dadurch **gekennzeichnet**, daß drei sich im wesentlichen vertikal nach oben erstreckende Arme (20) winkelmäßig versetzt an dem Ring (18) befestigt sind.

4. Flaschenständer-Kopf nach Anspruch 1,
dadurch **gekennzeichnet**, daß die Abstützung (12) eine Grundplatte (26) mit einer den Flaschenhals (15) aufnehmenden kreisförmigen Ausnehmung (28) und zumindest einen den Flaschenkörper (14) wenigstens teilweise umgreifenden Arm (20') aufweist.

5. Flaschenständer-Kopf nach Anspruch 4,
dadurch **gekennzeichnet**, daß mehrere Abstützungen (12) eine gemeinsame Grundplatte (26) aufweisen.

6. Flaschenständer-Kopf nach Anspruch 4 oder 5,
dadurch **gekennzeichnet**, daß je drei sich im wesentlichen vertikal nach oben ersteckende Arme (20') winkelmäßig versetzt um eine kreisförmige Ausnehmung (28) herum angeordnet sind.

7. Flaschenständer-Kopf nach einem der Ansprüche 2 bis 6,
dadurch **gekennzeichnet**, daß der oder die Arme (20, 20') einstückig ausgebildet sind.

8. Flaschenständer-Kopf nach einem der Ansprüche 2 bis 7,
dadurch **gekennzeichnet**, daß der oder die Arme (20, 20') federnd nachgiebig ausgebildet sind.

9. Flaschenständer-Kopf nach einem der vorhergehenden Ansprüche,
dadurch **gekennzeichnet**, daß mehrere Abstützungen (12) in einer rechtwinkelig zur Vertikalachse eines Flaschenständers (50) liegenden Ebene winkelmäßig versetzt angeordnet sind.

10. Flaschenständer-Kopf nach einem der vorhergehenden Ansprüche,
dadurch **gekennzeichnet,** daß zusätzlich mindestens ein Haken zum Aufhängen von in einen menschlichen Körper zu überführende Lösung enthaltenden Beuteln vorhanden ist.

11. Flaschenständer-Kopf gemäß Anspruch 1,
dadurch **gekennzeichnet,** daß der Kopf (10) zumindest zwei sich horizontal erstreckende Platten (54, 56) aufweist, die vertikal übereinander angeordnet sind, wobei die obere Platte (54) Löcher (60) aufweist, deren Durchmesser etwas größer ist als der Durchmesser eines Flaschenkörpers (14) und die untere Platte (56) Löcher aufweist (62), deren Durchmesser etwas grö0er ist als derjenige eines Flaschenhalses (15).

12. Flaschenständer-Kopf (10) gemäß Anspruch 1,
dadurch **gekennzeichnet,** daß zwei sich horizontal erstreckende Gitter (66, 68) vorgesehen sind, die vertikal übereinander angeordnet sind, wobei das obere Gitter (66) eine Maschenweite aufweist entsprechend dem Durchmesser eines Flaschenkörpers (14) und das untere Gitter (68) eine Maschenweite entsprechend dem Durchmesser eines Flaschenhalses (15).
